# EUROPEAN PATENT SPECIFICATION

(11) **EP 1 843 738 B1**
(45) Date of publication and mention of the grant of the patent: **31.03.2010**
(21) Application number: 06704561.7
(22) Date of filing: 12.01.2006
(51) Int. Cl.: A61K 8/19, A61K 8/49, A61Q 11/00

(54) **MULTIPHASE TOOTHPASTE COMPOSITION**
MEHRPHASIGE ZAHNPASTENZUSAMMENSETZUNG
COMPOSITION DE DENTIFRICE MULTIPHASE

(30) Priority: 04.02.2005 IN MU01182005; 08.04.2005 IN KO02882005
(43) Date of publication of application: 17.10.2007
(73) Proprietor: Unilever N.V., 3013 AL Rotterdam (NL); Unilever PLC, London EC4Y 0DY (GB)
(72) Inventor: BIJLANI, Nand Sanmukhdas, Mumbai 400 020 (IN); GREGORY, Donald Peter, Bebington, Wirral Merseyside CH 63 3JW (GB); GROVES, Brian, Bebington, Wirral Merseyside CH63 3JW (GB)
(74) Representative: James, Helen Sarah
(86) International application number: PCT/EP2006/000256
(87) International publication number: WO 2006/081926

(56) References cited:
- EP-A- 0 331 617
- DE-A1- 19 854 086
- US-A- 4 456 585
- US-A- 5 324 505

## Description

The present invention relates to a multiphase toothpaste composition comprising a clear phase and a core phase.

WO 99/01342 discloses apparatus for inserting plural materials into containers. The apparatus comprises a nozzle with a first hollow member and a second hollow member arranged inside the first hollow member. The nozzle is designed for directing the extrusion of multiple toothpaste phases into a toothpaste container with one phase being arranged within another.

Despite the prior art there remains the need for multiphase toothpaste compositions with improved visual impact and a cleaner appearance.

The present inventors have found that improved visual impact, a reduced 'dirty' look and better cleaner appearance is achieved by including a small amount of pigment into a visually clear outer phase. Accordingly and in a first aspect, the present invention provides a multiphase toothpaste according to claim 1.

By visually-clear is meant that the inner, first phase can be seen through the outer, second phase.

The first phase is disposed co-axially within the visually clear gel phase. By this is meant that the longitudinal axis of the ribbon as dispensed from the toothpaste container falls within the inner, first phase. Similarly, the general longitudinal axis of the toothpaste as stored within the toothpaste container falls within the inner, first phase as stored therein. Such alignment is understood to be judged by the eye and not mathematically.

When looking at a section of the dispensed ribbon end-on the inner phase may extend towards or up to the exterior surface of the ribbon. Such extension may be radial, spiral or abstract and, where it is radial or spiral it may be regular or irregular. Regular radial extensions are the most preferred to the consumer. In addition the core may, in cross section be of any shape, for example, star-shaped, square shaped, triangular, etc. These shaped cores are a real plus to the younger consumers. However, it is most preferred that the inner phase comprises no extensions and instead provides nothing more than a regular core to the ribbon. This regular shape is less fussy and provide clean lines to the product which reinforces the impression of cleanliness that toothpastes aim to provide.

The pigment is not water soluble.

Preferably, the outer phase comprises from 10 to 70 ppm and more preferably from 35 to 65 ppm of a pigment.

A preferred blue pigment is one chosen from the selection of Phthalocyanine Blue Pigments commercially available from Meilida Pigment Industry Co.

The various blue pigments include:
Phthalocyanine Blue BGS.
Phthalocyanine Blue BGNCF-03
Phthalocyanine Blue BGSP
Phthalocyanine Blue BRSH press cake
Phthalocyanine Blue BRX
Phthalocyanine Blue BRXH
Phthalocyanine Blue M-3000
Phthalocyanine Blue 94817D
Phthalocyanine Blue BGS-W
Phthalocyanine Blue BRS
Phthalocyanine Blue BRSS
Phthalocyanine Blue BRX press cake
Phthalocyanine Blue M-1000HF

The most preferred blue pigment is Phthalocyanine Blue Pigment CI No. 74260.

The preferred red pigments include Cosmetic Pink. The most preferred Cosmetic Pink is Cosmetic Pink CI-73360.

The preferred Green pigment is Phthalocyanine Green, preferably Phthalocyanine Green CI-74260.

In a preferred embodiment and when the inner phase of the toothpaste is white or blue, it is preferred that the pigment used at low levels in the outer phase is blue.

In a preferred embodiment and when the inner phase of the toothpaste is green, it is preferred that the pigment used at low levels in the outer phase is green.

In a preferred embodiment and when the inner phase of the toothpaste is red, it is preferred that the pigment used at low levels in the outer phase is red.

Such reduced levels of pigments markedly improve the perception of the inner phase within the outer phase. This reinforces the cleaning benefit provided by such toothpastes.

The phase is opaque. This adds a further benefit in that it creates a silvery effect at the interface between the two phases. This is an attractive effect for the consumer.

The inner phase of the toothpaste, when coloured, comprises from 0.01 to 0.2% by weight of the phase of a pigment. Preferably, the pigments are selected from those used for the outer phase. More preferably, the pigment is present in the inner phase at from 0.03 to 0.15% and most preferably around 0.08% by weight of the inner phase.

The opacity of the inner phase is provided by chalk or titanium dioxide.

The inner phase constitutes up to 25% by volume of the toothpaste. In using an inner first phase which constitutes up to 25% of the volume of the toothpaste composition an improved visual impact is obtained. Should the inner phase constitute much more than 25% by volume of the composition the inner phase appears to overwhelm the composition such that the outer phase is hardly noticed at all by the consumer. This is only the case when the outer phase is visually clear as it is in the present invention.

Preferably, the inner first phase constitutes from 11 to 20% and more preferably from 13 to 18% by volume of the toothpaste composition.

The first and second phases may be the same or different with regard to their principle components, i.e. thickeners, actives, structurants and abrasives. Where the first and second phases are essentially the same they may differ in minor components such as colours or flavours. Having two visually clear phases also provides an attractive composition for the consumer.

In a further preferred embodiment the outer, visually clear phase comprises abrasive silica. The particular abrasive silica used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the composition generally ranges from 5-60% by weight, usually 5-20% by weight.

In a preferred embodiment the inner, opaque phase comprises chalk, preferably fine ground natural chalk. The inner composition will preferably comprise chalk at from 10 to 60 % by weight of the phase.

The phases of the composition according to the invention are manufactured using standard processes. They may be extruded into a container for dispensing by equipment such as that discussed in detail in WO 99/01342, i.e. a coaxial nozzle assembly attached to standard equipment.

In a most preferred embodiment the phases according to the composition of the invention have viscosities as measured on a Brookfield RV DV-1 viscometer fitted with a Helipath stand at 25°C and 5rpm using a T-D spindle are from 150 000 Pa.s and 250 000 mPa.s. Such viscosities provide the best performance with regard to extrusion into the container and also from the container by the consumer. The phases within this viscosity range are much more stable, physically during extrusion, that other phases.

The toothpaste composition according to the present invention can comprise an agent selected from the group consisting of anti-carries agents, tooth whitening agents, anti-tartar agents, anti-malodour agents, anti-gingivitis agents and mixtures thereof.

The toothpaste composition will comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol); anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. Preferred abrasives are chalk and silica, more preferably fine ground natural chalk.

Humectants such as glycerol, sorbitol, propyleneglycol, xylitol, lactitol etc.; Preferably, the toothpaste's outer phase comprises from 55 to 70% by weight of the respective phase humectant. Preferably, this is 55 to 70% by weight of the phase sorbitol. The sorbitol will typically be present as a 70% aqueous solution.

binders and thickeners such as sodium carboxymethylcellulose, hydroxyethyl cellulose (Natrosol^{®}), xanthan gum, gum arabic etc. as well as synthetic polymers such as polyacrylates and carboxyvinyl polymers such as Carbopol^{®}; polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

In a second aspect the present invention provides a toothpaste container comprising a first and second phase as described in the first aspect of the invention stored within a tubular container, the container comprising a tubular body which is crimped at one end and comprises a dispensing assembly at the other through which the composition is extruded by the consumer.

Preferably, the tubular container is transparent or translucent so that the inner phase can be seen within the outer phase within the container.

An embodiment of the invention is now discussed in the nonlimiting example.

### EXAMPLES

This composition comprises a first phase and a second phase. The second phase is visually clear and the inner phase is opaque. The composition comprises 15% v/v of the first phase and 85% v/v of the second phase.

| **Ingredient** | **% (w/w) of the first phase** | **% (w/w) of the second phase** |
|---|---|---|
| water | 12, 48 | 12,73 |
| sorbitol (70%aq) | 63,00 | 63,00 |
| sodium fluoride | 0,32 | 0,32 |
| polyethylene glycol (PEG 32) | 4,00 | 4,00 |
| titanium dioxide | 0,50 | 0,00 |
| thickening silica | 9,00 | 9,25 |
| Abrasive silica | 8,00 | 8,00 |
| sodium carboxymethylcellulose | 0,90 | 0,90 |
| sodium lauryl sulphate | 1,80 | 1,80 |
| Phthalocyanine Blue Pigment CI No. 74260 | 0,08 | 40ppm |
| flavours | trace | trace |

## Claims

1. Multiphase toothpaste composition comprising a first opaque phase that constitutes up to 25% of the volume of the toothpaste and comprises chalk or titanium dioxide, disposed co-axially within a second, visually clear gel phase comprising from 1ppm to 100 ppm of a pigment that is not water soluble.

2. Multiphase toothpaste according to claim 1 comprising from 10 to 70 ppm of a pigment.

3. Multiphase toothpaste according to claim 1 or 2 comprising from 35 to 65 ppm of a pigment.

4. Multiphase toothpaste according to any preceding claim wherein the pigment is blue.

5. Multiphase toothpaste according to any preceding claim wherein the blue pigment is Phthalocyanine Blue Pigment.

6. Multiphase toothpaste according to any preceding claim wherein the blue pigment is Phthalocyanine Blue Pigment Cl No. 74260.

7. Multiphase toothpaste according to any of claims 1 to 3 wherein the pigment is red.

8. Multiphase toothpaste according to claim 7 wherein the pigment is Cosmetic Pink.

9. Multiphase toothpaste according to claim 7 wherein the pigment is Cosmetic Pink CI-73360.

10. Multiphase toothpaste according to any of claims 1 to 3 wherein the pigment is green.

11. Multiphase toothpaste according to claim 10 wherein the pigment is Phthalocyanine Green.

12. Multiphase toothpaste according to claim 11 wherein the pigment is Phthalocyanine Green CI-74260.

13. Multiphase toothpaste according to any preceding claim wherein the first phase constitutes from 11 to 20% by volume of the toothpaste composition.

14. Multiphase toothpaste composition according to any preceding claim wherein the first phase constitutes from 13 to 18% by volume of the toothpaste composition.

15. Multiphase toothpaste composition according to any preceding claim wherein the inner phase is coloured.

16. Multiphase toothpaste composition according to any preceding claim wherein the visually clear gel phase comprises abrasive silica.

17. Multiphase toothpaste composition according to any preceding claim wherein the inner phase comprises chalk as an abrasive.

18. Multiphase toothpaste composition according to any preceding claim wherein the visually clear gel phase comprises an abrasive silica having a Refractive index of from 1.41 to 1.47

## Patentansprüche

1. Mehrphasige Zahnpasta-Zusammensetzung, umfassend eine erste opake Phase, die bis zu 25 % des Volumens der Zahnpasta bildet und Kreide oder Titandioxid umfasst, die koaxial innerhalb einer zweiten, visuell klaren Gelphase, die 1 ppm bis 100 ppm eines Pigments, das nicht wasserlöslich ist, umfasst, angeordnet ist.

2. Mehrphasige Zahnpasta gemäß Anspruch 1, die 10 bis 70 ppm eines Pigments umfasst.

3. Mehrphasige Zahnpasta gemäß Anspruch 1 oder 2, die 35 bis 65 ppm eines Pigments umfasst.

4. Mehrphasige Zahnpasta gemäß einem vorangehenden Anspruch, in der das Pigment blau ist.

5. Mehrphasige Zahnpasta gemäß einem vorangehenden Anspruch, wobei das blaue Pigment Phthalocyaninblau-Pigment ist.

6. Mehrphasige Zahnpasta gemäß einem vorangehenden Anspruch, wobei das blaue Pigment Phthalocyaninblau-Pigment CI No. 74260 ist.

7. Mehrphasige Zahnpasta gemäß einem der Ansprüche 1 bis 3, wobei das Pigment rot ist.

8. Mehrphasige Zahnpasta gemäß Anspruch 7, wobei das Pigment Cosmetic-Pink ist.

9. Mehrphasige Zahnpasta gemäß Anspruch 7, wobei das Pigment Cosmetic-Pink CI-73360 ist.

10. Mehrphasige Zahnpasta gemäß einem der Ansprüche 1 bis 3, wobei das Pigment grün ist.

11. Mehrphasige Zahnpasta gemäß Anspruch 10, wobei das Pigment Phthalocyaningrün ist.

12. Mehrphasige Zahnpasta gemäß Anspruch 11, wobei das Pigment Phthalocyaningrün CI-74260 ist.

13. Mehrphasige Zahnpasta gemäß einem der vorangehenden Ansprüche, wobei die erste Phase 11 bis 20 Volumen-% der Zahnpasta-Zusammensetzung ausmacht.

14. Mehrphasige Zahnpasta-Zusammensetzung gemäß einem vorangehenden Anspruch, wobei die erste Phase 13 bis 18 Volumen-% der Zahnpasta-Zusammensetzung ausmacht.

15. Mehrphasige Zahnpasta-Zusammensetzung gemäß einem vorangehenden Anspruch, wobei die innere Phase gefärbt ist.

16. Mehrphasige Zahnpasta-Zusammensetzung gemäß einem vorangehenden Anspruch, wobei die visuelle klare Gelphase Silica als Schleifmittel umfasst.

17. Mehrphasige Zahnpasta-Zusammensetzung gemäß einem vorangehenden Anspruch, wobei die innere Phase Kalk als Schleifmittel umfasst.

18. Mehrphasige Zahnpasta-Zusammensetzung gemäß einem vorangehenden Anspruch, wobei die visuell klare Gelphase ein Silica als Schleifmittel umfasst, das einen Brechungsindex von 1,41 bis 1,47 hat.

## Revendications

1. Composition de dentifrice multiphase comprenant une première phase opaque qui constitue jusqu'à 25 % du volume du dentifrice et comprend de la craie ou du dioxyde de titane, disposée coaxialement au sein d'une deuxième phase de gel visuellement claire, comprenant de 1 ppm à 100 ppm d'un pigment qui n'est pas soluble dans l'eau.

2. Dentifrice multiphase selon la revendication 1, comprenant de 10 à 70 ppm d'un pigment.

3. Dentifrice multiphase selon la revendication 1 ou 2, comprenant de 35 à 65 ppm d'un pigment.

4. Dentifrice multiphase selon l'une quelconque des revendications précédentes, dans lequel le pigment est bleu.

5. Dentifrice multiphase selon l'une quelconque des revendications précédentes, dans lequel le pigment bleu est un pigment bleu de phtalocyanine.

6. Dentifrice multiphase selon l'une quelconque des revendications précédentes, dans lequel le pigment bleu est le pigment bleu de phtalocyanine CI n° 74260.

7. Dentifrice multiphase selon l'une quelconque des revendications 1 à 3, dans lequel le pigment est rouge.

8. Dentifrice multiphase selon la revendication 7, dans lequel le pigment est le rose cosmétique.

9. Dentifrice multiphase selon la revendication 7, dans lequel le pigment est le rose cosmétique CI-73360.

10. Dentifrice multiphase selon l'une quelconque des revendications 1 à 3, dans lequel le pigment est vert.

11. Dentifrice multiphase selon la revendication 10, dans lequel le pigment est le vert de phtalocyanine.

12. Dentifrice multiphase selon la revendication 11, dans lequel le pigment est le vert de phtalocyanine CI-74260.

13. Dentifrice multiphase selon l'une quelconque des revendications précédentes, dans lequel la première phase constitue de 11 à 20 % en volume de la composition de dentifrice.

14. Composition de dentifrice multiphase selon l'une quelconque des revendications précédentes, dans laquelle la première phase constitue de 13 à 18 % en volume de la composition de dentifrice.

15. Composition de dentifrice multiphase selon l'une quelconque des revendications précédentes, dans laquelle la phase interne est colorée.

16. Composition de dentifrice multiphase selon l'une quelconque des revendications précédentes, dans laquelle la phase de gel visuellement claire comprend de la silice abrasive.

17. Composition de dentifrice multiphase selon l'une quelconque des revendications précédentes, dans laquelle la phase interne comprend de la craie en tant qu'abrasif.

18. Composition de dentifrice multiphase selon l'une quelconque des revendications précédentes, dans laquelle la phase de gel visuellement claire comprend une silice abrasive ayant un indice de réfraction de 1,41 à 1,47.
